# EUROPEAN PATENT APPLICATION

(11) **EP 2 995 621 A1**
(43) Date of publication of application: **16.03.2016**
(21) Application number: 14184307.8
(22) Date of filing: 10.09.2014
(51) Int. Cl.: C07K 7/06, C07K 16/00, C07K 14/31, C07K 14/315

(54) **Ligands and methods for isolating or removing proteins**

(71) Applicant: Previpharma AG, 6300 Zug (CH)
(72) Inventor: KIESSIG, Stephan T., 69168 Wiesloch (DE); MÜLLER, Dirk, 68165 Mannheim (DE)
(74) Representative: Held, Stephan

(57) **Abstract**

The present disclosure relates generally to sorbents for binding proteins from blood plasma comprising a solid carrier material and an oligopeptide immobilized thereon, which selectively binds the protein from the blood plasma, and to a method for separating autologous proteins from blood plasma wherein the blood plasma is brought in contact with a corresponding sorbent, the desired protein is allowed to bind to the sorbent, the sorbent is separated from the plasma and the protein is subsequently separated from the sorbent. Corresponding sorbents and method allows for the selective isolation of specific blood plasma proteins directly from the blood plasma without having to conduct a tedious fractionation. This significantly improves the protein yield and provides a highly effective isolation of blood plasma protein constituents such as IgG, albumin, factor V and factor VIII in a simple, cheap and time-efficient process.

## Description

### Field of the Invention

The present invention relates to ligands and methods for isolating and obtaining or removal or binding of proteins, in particular proteins autologous to the blood or other body fluids from which they are isolated, including for example immunoglobulins (e.g., IgG), Albumin, specific antibodies, coagulation factors, or coagulation inhibitors, which can be used for the treatment of patients suffering from immunodeficiency or other disorders. The present invention also relates to methods for obtaining blood plasma proteins for the treatment of diseases or disorders by isolating these proteins from a donor and returning at least a portion of the protein depleted blood plasma to the donor and methods for reducing blood plasma protein levels of autoimmune disease associated proteins in a patient suffering from such disease.

### Background of the invention

Immunoglobulins isolated from blood plasma (also known as antibodies) are specialised antigen binding proteins that are found in blood or other bodily fluids of vertebrates and act primarily as a defence against an invasion by foreign substances such as bacteria and viruses. Antibodies can come in different varieties known as isotypes. In mammals there are five antibody isotypes known as IgA, IgD, IgE, IgG and IgM, each of which comprises different effector functions. IgG provides the majority of antibody-based immunity against invading pathogens.

Numerous diseases and/or disorders are associated with primary or secondary immunodeficiencies (e.g., a reduction or defect in the production of a sufficient amount of IgG and/or IgM and/or IgA) or a defect in immune modulation (e.g., chronic inflammation). Additionally or alternatively, such treatments may also include administration of immunoglobulins to an immuno-compromised subject. However, methods for obtaining immunoglobulins from a donor require several processing steps at one or more facilities that are often at a different location from the donor and recipient delaying transfusion and leading to increased costs.

The most established process to isolate immunoglobulins, coagulation factors, inhibitors, or albumin from blood plasma is the Cohn fractionation (cf. e.g. Harris, J. R. Blood Separation and Plasma Fractionation. Wiley-Liss. 1991). This process was developed in the 1940ies and basically involves a sequential precipitation of different blood plasma protein fractions at different temperatures, pH and ethanol concentrations. While the Cohn process is still used today, it suffers from significant loss of albumin to be isolated due to the sequential precipitation. This makes the process both relatively tedious and low yielding.

The original Cohn process since the 1940ies has been modified to optimize e.g. the yield of albumin for example by Gerlough in 1955, Kistler and Nitschmann or Hao in 1975 (cf. Graham, J.M., Rickwood, D. Subcellular Fractionation, a Practical Approach. Oxford University Press. 1997). However, all of these optimizations did not deviate from the basic concept of the Cohn Process and still involve a fractional precipitation with ethanol to provide moderate yields of the different plasma proteins (e.g. albumin) relative to the total amount of the protein of interest in the blood plasma. In some of these processes these plasma is treated with ethanol at elevated temperatures of 68 °C to deactivate viruses in the blood and to further increase the yield of albumin. Even though, heat treatment of blood plasma results in a substantial risk of denaturation of the albumin leading to unwanted antigens in the product. Another draw-back of heat ethanol fractionation is that the heat treatment vessels required are relatively expensive which diminishes the advantage of higher albumin recovery relative to cold ethanol extraction processes. General disadvantages of all of the above processes is that ethanol are required for the extraction which results in a significant risk due to the flammability of ethanol. In addition, the use of ethanol makes it impossible that the plasma constituents which are not needed can be returned to the donor.

A relatively recent development in the field of protein isolation from blood plasma is the use solid support immobilized proteins which selectively bind for example to immunoglobulin IgG. In this respect, US 2010/0158893 A1 describes the selective binding of IgG to a solid support modified with protein A by directly contacting the modified solid support with blood plasma. Upon contact, the solid support absorbs the IgG in the plasma and by changing e.g., the pH, salt environment or temperature, the IgG can then be re-eluted from the solid support. The obtained IgG can subsequently either directly be administered to a recipient or can be subjected to further purification through conventional techniques. A down-side of the approach described in US 2010/0158893 A1 is however, that protein A being a protein of the 441 amino acids is relatively costly to prepare and difficult to handle. This results in a significant cost factor for the preparation of the absorbent materials of US 2010/0158893, in particular, when the support has to be discarded after use for hygienic reasons.

Affinity chromatography has also been implemented for isolating and removing pathogenic blood constituents from whole blood. For Example Gregori et al. describe in Transfusion 2006, vol. 46, p. 1152 to 1161, that small molecules and peptides comprising 3 to 7 amino acids (see also WO 2004/050851 A1), which had been immobilized on chromatography resin beads, can be used to remove the PK-specific form of prion protein, which is associated with (new) variant Creutzfeld Jacob disease (vCJD) from whole blood samples. In this study, the affinity ligands for the prion protein were identified from combinatorial libraries comprising millions compounds.

Thus far, however no methodology is available, which allows for the isolation of specific proteins, in particular proteins, which naturally occur in mammals and are present in their native form, from blood in a cheap and easy manner. Therefore, there remains a need for a method for isolating specific proteins in blood plasma with high yields, to thus overcome the disadvantages of the above-described Cohn process. Moreover, there remains a need for an easy and effective method to immobilize and capture proteins from blood plasma, which leaves the residual constituents in the blood plasma substantially unaffected to allow for returning the residual plasma to a donor. Such method would also have significant advantages for therapeutic and preparative apheresis as well as in dialysis and would allow for an effective and specific elimination of pathogen proteins from the blood of a patient without affecting other constituents. The present application addresses these needs.

### Brief description of the drawings

Figure 1 shows a correlation of the ligand concentration with its absorption on an IgG (lower curves) or albumin (upper curves) covered ELISA plate for peptide 3.
Figure 2 shows the absorption of IgG on an ELISA plate coated with peptide 1 for different peptide 1/conjugate concentrations.
Figure 3 shows the absorption of IgG on an ELISA plate coated with peptide 1 for different peptide 2/conjugate concentrations.
Figure 4 shows the absorption of IgG on an ELISA plate coated with peptide 1 for different peptide 3/conjugate concentrations.

### Detailed description

In a first aspect, the present application relates to a sorbent for binding proteins from blood plasma, comprising a solid carrier material and an oligopeptide immobilized thereon, which selectively binds a protein from the blood plasma. Preferably, the protein is a protein autologous to the blood plasma, more preferably a native protein which is autologous to the plasma, i.e., a protein, which has its regular native folding..

"Autologous", as this term is used in the context of the present application means, that the protein is naturally occurring in the healthy organism, i.e., it is produced by the healthy organism and is regularly present in the healthy organism. Hence, e.g. viruses and virus parts are not encompassed by the term "autologous" as they regularly stem from viruses which are introduced into an organism from the exterior.

The term "native" is meant to exclude misfolded proteins such as for example prion protein, which has a folding associated with the induction of Creutzfeld Jacob's disease.

For the oligopeptide, it is preferred, that it has less than 70 and 7 or more, in particluar 8 to 50, more preferably 10 to 30 and most preferably 12 to 20 amino acids. If the oligopeptide has more than 70 amino acids, the preparation and handling of the oligopeptide becomes much more difficult and the oligopeptide thus becomes much more expensive to prepare. If the oligopeptide has less than 7 amino acids, a selectivity (affinity) for a specific protein or a group of similar proteins in the blood plasma can no longer be ensured.

With regard to the sorbent, it is further preferred that the oligopeptide selectively binds to immunoglobulins, in particular immunoglobulin G (IgG), factor V, factor VIII or albumin.

If the oligopeptide selectively binds to IgG, the IgG is preferably a polyclonal IgG. The oligopeptide can be selected also in a way that only specific Immunoglobulins will be recognized and bound (e.g. anti-tetanus IgG for normal and/or hyper immune plasma, anti-D immunoglobulin [against rhesus factor Rh(d)] from normal or hyperimmune plasma, anti-hepatitis B immunoglobulin from normal or hyperimmune plasma, anti-hepatitis C immunoglobulin from normal or hyperimmune plasma or other specific antibodies which recognize bacterial or viral antigens.

On the other hand, it is preferred that the oligopeptide immobilized on the solid material is not an oligopeptide which binds to prion protein variants. More preferably, the oligopeptide is not an oligopeptide which binds to prion protein variants which are associated with the induction of Creutzfeld Jacob's disease.

In a highly preferred embodiment, the oligopeptide comprises a sequence selected from
i) KEDGNKPGKED
ii) KEDGNKP
iii) DTVNDIAKANGTTAD
iv) LETEKQISEASRKSL
v) DLTAAAVADTVA
vi) DAPTEPEKPE
vii) KQISD/EASR
viii) LEKLELDYLKKL
ix) SQTPDTKPGNK
x) LSRDLEASRAA
or a sequence having at least 80% sequence identity with the above indicated i) to x) and having a binding affinity toward IgG. The designation D/E means that this amino acid can either be D or E.

In another highly preferred embodiment, the oligopeptide comprises a sequence selected from
i) PGKEDGNKPGKEDGN
ii) TADLTAAAVADTVAA
iii) PGDAPTEPEKPEASI
iv) KEDKQISDASRQGLS
v) QGKLEKLELDYLKKL
vi) SDSQTPDTKPGNKAV
vii) KEDGNKPGK
viii) DKQISDASR
ix) KSLSRDLEASRAAKK
or a sequence having at least 80% sequence identity with the above indicated i) to vi) and having a binding affinity toward IgG.

The term "percentage sequence identity" is intended to denote a percentage of amino acid residues which are identical between the two sequences to be compared, obtained after the best alignment, this percentage being purely statistical and the differences between the two sequences being distributed random and over their entire length. Sequence comparisons between two amino acid sequences are conventionally carried out by comparing the sequences after having aligned them optimally, said comparison being carried out by segment or by "window of comparison" in order to identify and compare local regions of sequence similarity. The optimal alignment of the sequences for comparison may be produced, besides manually, by means of the local homology algorithm of Smith and Waterman (1981) Ad. App. Math., 2:482, by means of the local homology algorithm by Neddleman and Wunsch (1970), J. Mol. Biol. 48:443, by means of the similarity search method by Pearson and Lipman (1988), Proc. Natl. Acad. Sci, USA, 85:4222, or by means of computer programs which use these algorithms. It is noteworthy, that the sequence identity as used in this application does not account for the difference between L and D-amino acids so that a sequence consisting exclusively of D-amino acid would be 100% sequence identical to the same sequence consisting only of L-amino acids.

The percentage identity between two amino acid sequences is determined by comparing these two sequences in light an optimal manner in which an amino acid sequence to be compared may comprise additions or deletions compared to the reference sequence for optimal alignment between these two sequences. The percentage identity is calculated by determining the number of identical positions for which the amino acid residue is identical between the two sequences, by dividing this by the number of positions compared and by multiplying the result obtained by 100 so as to obtain the percentage identity between the two sequences.

The binding affinity can be determined by a test as indicated in Example 2, wherein an oligopeptide must have more than an unspecific binding towards the investigated protein.

For the sequence having at least 80% sequence identity with the above indicated i) to x) it is preferred that they have at least 90 % sequence identity.

It is in particular preferred with regard to IgG binding oligopeptides, that they comprises a sequence selected from i) KEDGNKPGKED, ii) KEDGNKPGK and iii) DTVNDIAKANGTTAD.

In an alternative embodiment, where the oligopeptide selectively binds to factor V or factor VIII, it is preferred that the oligopeptide comprises a sequence selected from
i) LEGYDLRRWEKWE
ii) HNYGVYTKVSRYL
iii) GDSGGPMVASFHG
iv) FVHPNYSKSTTDNDIALLH
v) TGWGYHSSREKEA
or a sequence having at least 80% sequence identity with the above indicated i) to v) and having a binding affinity towards factor V or factor VIII. Since the binding affinity of the above sequences iv) and v) is weaker than the binding affinity of the sequences in i) to iii), the oligopeptides preferably comprise a sequence as indicated in i) to iii).

If the purpose of the oligopeptide is to immobilize only factor V, it is most preferred that the oligopeptide comprises a sequence HNYGVYTKVSRYL as this sequence has been identified as not significantly binding to factor VIII. If instead, it is the intention to selectively immobilize factor VIII, the sequence is most preferably GDSGGPMVASFHG as this sequence has been identified to not significantly bind to factor V thus having high selectivity for factor VIII. If in turn it is the intention to immobilize both factors V and VIII, it is most preferred that the oligopeptide comprises a sequence LEGYDLRRWEKWE, because this sequence binds equally strong to both factor V and factor VIII.

Also for the above sequences having at least 80% sequence identity with the above indicated i) to v) it is preferred that they have at least 90 % sequence identity.

Modifications, which lead to less than 100 % sequence identity, regularly include those, which do not affect the binding of the peptide towards the target protein but which improve the stability of the peptides towards acidic or alkaline hydrolysis, and the degradation by proteases and peptidases. An example of a non-natural amino acide suitable for this purpose is L-citrullin. In the practice of the present application, it is preferred that the oligopeptide comprises at least one such modification.

Moreover, since D-amino acid containing peptides are known to have higher stability towards degradation by proteases and peptidases and since blood serum regularly contains serine proteases, it is preferred that the oligopeptides contain at least one D-amino acid to improve the stability towards protease and peptidase degradation.

As concerns the solid carrier material, there are no significant limitations except that the solid material should not induce coagulation or a reaction with other proteins and constituents of the blood plasma. For example, it is possible and in some cases advantageous, if the solid carrier material is latex material, more preferably a magnetic latex material, as this allows to handle the latex for example with a magnet without having to use filtration equipment. Other materials which can be used as a solid carrier material include agarose carriers (e.g. Sepharose), dextran based carriers (e.g Sephadex), glass based carriers such as beads or slides, Polyvinyl-ether based carriers, acrylic polymer based carriers, Polyacrylamide carriers, Nitrocellulose, Nylon or PVDF The carrier could behave a variety of shapes and forms such as particles, columnar (e.g. in the form of a filter), cylindrical, etc. The term solid in the context of solid carrier material encompasses both gels and particulate materials.

As indicated above, the oligopeptide is immobilized on the solid carrier material. For this purpose, essentially all methods known in the art to immobilize oligopeptides on solid supports can be used. For example the oligopeptide could be modified with a maleimide and the solid support could comprises thiol groups to allow for an addition of the thiol to the maleimid. Another effective method to immobilize oligopeptides on a solid material is using a streptavidin biotin interaction or hydrophobic interactions. To assure, that the oligopeptide does not significantly interact with the streptavidin, it is advantageous in this regard, if the streptavidin is bound to the solid carrier material, while the oligopeptide has a biotin label to enable binding of the oligopeptide to the solid carrier material. A commercially available carrier material available for this purpose is streptavidin coated agarose, which is available e.g. from GenScript. In the context of the present application it is preferred however, that the oligopeptide is immobilized on the solid carrier material in an irreversible manner, such as e.g. by covalently binding the oligopeptide to the solid carrier material.

A second aspect of the present invention relates to a container having an inlet and an outlet for fluids, which comprises a filling of a sorbent as described above. For such container, which can e.g. be filled with particles, or can have the form of a filter containing the respective oligopeptide, it is preferred, that the outlet is positioned on the side opposite to the inlet of the container to ensure, that the fluid gets in good contact with the sorbent and has to move through the sorbent to allow for an effective and efficient binding of desired proteins to the sorbent. Regarding the shape and dimensions of the container, there are no significant limitations, however, it is preferred if the container has a columnar shape and preferably is in form of a cartridge, more preferably a disposable cartridge. Moreover, it is preferred if the container has means at the inlet and outlet, which prevent the sorbent to be eluted from the container. An example of such means is a filter or a grid having a pore or mesh size, which is smaller than the size of the solid carrier material.

A further embodiment of the present application is directed at oligopeptides having less than 70, preferably 8 to 50, more preferably 10 to 30, and in particular 12 to 20 amino acids which comprise a sequence selected from
i) KEDGNKPGKED
ii) KEDGNKP
iii) DTVNDIAKANGTTAD
iv) LETEKQISEASRKSL
v) DLTAAAVADTVA
vi) DAPTEPEKPE
vii) KQISD/EASR
viii) LEKLELDYLKKL
ix) SQTPDTKPGNK
x) LSRDLEASRAA
xi) LEGYDLRRWEKWE
xii) HNYGVYTKVSRYL
xiii) GDSGGPMVASFHG
xiv) FVHPNYSKSTTDNDIALLH
xv) TGWGYHSSREKEA
or a sequence having at least 80% sequence identity with the above indicated i) to xv) and having a binding affinity toward IgG, factor V or factor VIII. The designation D/E means that this amino acid can either be D or E.

Another embodiment of the present application is directed at oligopeptides having less than 70, preferably 8 to 50, more preferably 10 to 30, and in particular 12 to 20 amino acids which comprise a sequence selected from
i) PGKEDGNKPGKEDGN
ii) TADLTAAAVADTVAA
iii) PGDAPTEPEKPEASI
iv) KEDKQISDASRQGLS
v) QGKLEKLELDYLKKL
vi) SDSQTPDTKPGNKAV
vii) KEDGNKPGK
viii) DKQISDASR
ix) KSLSRDLEASRAAKK
or a sequence having at least 80% sequence identity with the above indicated i) to ix) and having a binding affinity toward IgG.

The above described oligopeptides, can also be used with advantage in a context other than the above described sorbents and containers, namely in the context of diagnostics. Thus, the present application also encompasses the use of the above mentioned oligopeptides for the detection of the presence or absence of IgG, in particular specific IgG, factor V or factor VIII in an analyte. Such use can e.g. be implemented by immobilizing the oligopeptides on a solid support, then bringing the solid support in contact with the analyte to allow for a binding of the protein of interest to the oligopeptide, washing off any non-bound analyte components and finally adding a marker for the protein of interest to investigate whether the analyte contained this protein. In another embodiment, the analyte could be brought in contact with an antibody, which selectively binds a protein of interest, and then an oligopeptide as indicated above, which is modifies with e.g. a fluorescent tag, could be used as the marker.

In a yet further embodiment, the present application is directed at a method of separating a protein from blood plasma, cell culture supernatants and other protein preparations comprising the steps of
i) bringing the blood plasma in contact with a sorbent as described above under conditions which allow for binding of at least one protein from the blood plasma to the sorbent,
ii) separating the sorbent from the blood plasma, and
iii) subsequently separating the bound protein from the sorbent.

Preferably, the protein to be separated in this method is an autologous protein according to the above definition, more preferably an autologous native protein.

As indicated in the above, the source, from which the protein is to be isolated, is not decisive for the method, as long as the sorbent is brought in contact with a fluid, preferably with an aqueous solution comprising a protein to be immobilized on the sorbent and further proteins and/or constituents of a nourishing medium for cells.

In the context of the above method, it is further preferred that the sorbent is subjected to washing after step i) and before step iii) under conditions where the protein remains bound to the protein. This step helps to reduce the number of plasma proteins not bound to the solid carrier material or plasma protein unspecifically bound to the solid carrier material resulting in a more highly pure product. A suitable washing agent is a buffer such as PBS buffer.

The above method can further advantageously be extended by subjecting the protein which has been separated from the sorbent after step iii) to further purification to remove remaining impurities. A suitable further purification for an IgG isolate from blood plasma is for example described in WO 2014/076199 A1, the disclosure of which is herewith incorporated by reference.

It is possible to separate the bound protein from the sorbent by removing the sorbent from the system used in the method and to elute the protein by change of the pH, the flow rate or the salt concentration. If the sorbent is used in form of a cartridge, the protein can e.g. be eluted by slowly conveying an appropriate buffer through the cartridge and by collecting the eluate in a container.

A yet further embodiment of the present application relates to a method for separating an autologous protein from blood plasma, comprising the steps of
i) conveying blood from a donor to a first separation device, which separates the blood plasma from the blood,
ii) conveying the blood plasma to a second separation device, at which it is brought in contact with a sorbent as described above under conditions which allow for binding of at least one protein from the blood plasma to the sorbent, and
iii) subsequently collecting the blood plasma in a container.

The plasma collected in a container can be further processed by, e.g., cryoprecipitation or the Cohn process as described above to isolate further constituents in the blood plasma. In addition, it is possible to convey the plasma to a third or further separation devices containing a sorbent as described above, which is selective for a different blood plasma protein than the sorbent in the second separation device. Further, the blood plasma can also be conveyed to an affinity sorbent which is capable to remove virus components, such as components from HIV, emerging viruses, or prion proteins form the blood serum.

In an embodiment alternative to the above, the present application relates to a method for separating an autologous protein from blood plasma comprising the steps of
i) conveying blood from a donor to a first separation device, which separates the blood plasma from the blood,
ii) conveying the blood plasma to a second separation device, at which it is brought in contact with a sorbent as described above under conditions which allow for binding of at least one protein from the blood plasma to the sorbent, and
iii) subsequently returning at least a portion of the blood plasma to the donor.

For both of the afore-mentioned methods, the protein immobilized on the sorbent can be released therefrom, preferably by subjecting the sorbent to conditions which result in a release of the bound protein. This can either be done in an offline manner, i.e., in that the flow of the blood plasma onto the separation device in which the blood plasma is brought in contact with the sorbent is stopped, the separation device is removed from the system, optionally shipped to a further processing facility and then subjected to the conditions to allow for release of the bound protein. The protein can then be collected and further purified, e.g., in the case of IgG by polishing according to WO 2014/076199 as described above.

It is however also possible to release the bound protein in an online manner, e.g., by using a system which has a first fluid circuit to convey the blood and blood plasma to the second separation device and a second fluid circuit to convey a fluid effecting the release of the bound protein from the sorbent and to direct the released protein to a container. For operating such system in an "online manner" the flow of blood plasma onto the sorbent would have to be temporarily stopped, and while the flow of the plasma is stopped, the fluid in the second circuit would have to be directed to the sorbent to affect protein release. The released protein could then be directed to an individual container to capture the protein. The online manner has the advantages, that the donor has not to be disconnected from the extraction system to extract and separate the autologous protein from the blood plasma and that the sorbent can be re-used even in the course of isolating a specific protein from the blood plasma of a single donor.

Moreover, releasing the bound protein in an online manner has the further advantage that it becomes possible to administer the isolated protein directly to a subject or a patient which is in need of additional isolated protein by methods as described in US 2010/0158893 A1, instead of capturing the protein in a container. In this manner, the time between the isolation of the protein from the donor to the protein being administered to a patient can significantly be reduced substantially reducing the risk of protein degradation and contamination.

In a yet further embodiment, the present invention relates to a method for depleting an autologous protein from blood plasma of a patient comprising the steps of
i) conveying blood from a patient to a first separation device, which separates the blood plasma from the blood,
ii) conveying the blood plasma to a second separation device, at which it is brought in contact with a sorbent as described above under conditions which allow for binding of at least one protein from the blood plasma to the sorbent, and
iii) subsequently returning at least a portion of the blood plasma to the donor,
wherein the patient suffers from an overproduction of the protein to be removed in step ii).

This method can also be describes as a therapeutic apheresis. The method can further be improved if in the course of or after the steps i) to iii) above the bound plasma is released form the sorbent by subjection the sorbent to conditions as indicated above. Thus, the sorbent can be regenerated to allow for a further use.

The above-described methods can advantageously be developed further by bringing the blood plasma in contact with sorbents, which are selective binders for proteins other than the protein which has been removed from the blood serum in the second separation device. These sorbents could be arranged in a consecutive manner, e.g. in than the blood serum is passed through a sequence of several cartridges comprising each a sorbent selective for a different protein. In this manner blood plasma proteins such as IgG, factor V, factor VIII and albumincould be individually isolated from a single stream of blood serum by using the affinity ligands as described above. In addition, it is possible to use mixtures of more than one sorbent as described above, which, however, is less favoured in the practice of the invention as it provides mixtures of the targeted proteins which have to be discarded or separated in a further step.

The method is however not limited to these proteins, so that it would also be possible to isolate proteins such as pro-inflammatoric cytokines and other unwanted proteins from the blood plasma. In addition, the blood plasma could be brought in contact with conventional sorbents which are selective for prion proteins, in particular infective prion proteins (as decribed e.g. in Gregori et al. Transfusion 2006, vol. 46, p. 1152 to 1161), or viruses (as described e.g. in Tullis et al. Blood Purif. 2003, 21(1), p. 58-63).to allow for their removal from the blood plasma before or after the blood plasma has been brought in contact with the sorbent according to the invention as described above.

In the context of the method described above, where at least a portion of blood plasma is returned to the donor, it is in addition possible to use different sorbents selective for coagulation factors, activated proteases, pro-inflammatoric cytokines and other unwanted proteins thus providing an effective and time-efficient system for therapeutic apheresis and dialysis.

Finally it is noted, that the embodiments describes as preferred in the context of the sorbents or containers as likewise preferred in the context of all of the described methods.

In the following, the present application will be illustrated by means of Examples which should however not be considered as being limiting to the scope of the herein described invention.

### Examples

Identification of peptide antigens selective for IgG, factor V and factor VIII

To identify oligopeptide candidates for selective binding to IgG, a peptide array was done with F(ab')2 goat anti-human IgG (H+L) conjugated to DYLight680 antibody at a dilution of 1:5000 to investigate background interactions with the protein derived peptides that could interfere with the main assays. Subsequent incubation of the peptide microarrays with a polyvalent human IgG serum 375 at dilutions of 1:1000 and 1:100 in incubation buffer was followed by staining with the secondary antibody and by readout at a scanning intensity of 7 (red). HA and Flag control peptides framing the peptide arrays were finally stained as internal quality control to confirm the assay quality and the peptide microarray integrity (scanning intensities red/green: 7/7).

Quantification of spot intensities and peptide annotation were done with PepSlide^{®} analyser. A software algorithm breaks down fluorescence intensities of each spot into the raw, foreground and background signal and calculates the standard deviation of foreground median intensities. Based on average foreground median intensities, intensity maps were generated and binders in the peptide maps were highlighted by an intensity colour code with red for high and white for low spot intensities.

Further, the average spot intensities of all assays were plotted against the linked protein sequences from the N-terminus to the C-terminus of the investigated Protein templates to visualize overall spot intensities and signal to noise ratios. The intensity plots were correlated with this peptide and intensity maps as well as with visual inspection of the microarray scans to identify peptides and consensus motives that interacted with their polyvalent serum sample.

After 10 min pre-swelling in standard buffer (PBS, pH 7.4 to 0.05% Tween 20) and 20 minutes in blocking buffer (Rockland blocking buffer MP-070), one of the peptide arrays was initially incubated with the secondary F(ab')2 goat anti-human IgG (H+L) conjugated to DYLight680 antibody at a dilution of 1 to 5000 for 30 minutes at room temperature to analyse background interactions with the antigen-derived peptides. At scanning intensity of 7, no background was observed due to non-specific binding of the secondary antibody.

Two different peptide microarray copies were then incubated with polyvalent human an IgG serum 375 at dilutions of 1:1000 and 1:100 in incubation buffer (PBS, pH 7.4 with 0.05% Tween 20 and 10% blocking buffer). After each incubation, staining with secondary F(ab')2 goat anti-human IgG (H+L) conjugated to DYLight680 antibody was followed by readout at a scanning intensity of 7. On both peptide arrays, a number of well-defined interactions with clear epitope like spot patterns were observed which were formed by rows of neighboured peptides with a consensus motif, and a very complex response in the microarray area covered by protein A. Due to the higher serum concentration, the signal to noise ratios of the assays at a dilution of 1:100 was remarkably increased. The final staining of the HA and Flag control peptides framing the peptide arrays gave rise to the expected and well-defined spot pattern and validated the overall peptide microarray integrity.

In this manner, the sequences of Protein A, Protein G, Protein H and FcRn were investigated for peptides binding to IgG. In addition, the sequence of Protein C was investigated for peptides binding to factor V and factor VIII.

### Example 1:

Biotin-labelled peptides 1 to 3 as shown below were immobilized on latex particles.
Peptide 1 bio-X-GNKPGKEDGNKPGKEDGNKPG
Peptide 2 bio-X-QQQLITIKQISEASRKSLSRD
Peptide 3 bio-X-KPGDTVNDIAKANGTTADKIA.

In these peptides, "bio" designate biotin and X designates a linker with the sequence GSGSGSG.

For the immobilization a streptavidin coated latex was used. The synthesis of the modified latex particles was accomplished as follows:

First, the latex particles were washed five times with 2 ml PBS buffer. Then, the particles were allowed to settle for 5 minutes before a magnet was attached to the flask. The peptide was diluted in PBS buffer to a final concentration of 1 mg/ml, and the peptide solution was added to the latex particles and left over night at a temperature of 4 to 8°C. After a magnet had been attached to the flask, the supernatant protein solution was removed and the latex particles were washed five times with 0.5 ml PBS buffer.

Subsequently, it was evaluated whether IgG can be bound to and released from the thus prepared modified latex. For this purpose, 10 mg of the modified latex particles were mixed with 400 µl of an IgG solution containing 0.22 mg/ml IgG. After allowing the mixture to react at room temperature for one hour, the supernatant solution was removed from the latex beads and the beads were washed to remove any non-bound IgG. The beads were then evaluated for bound IgG.

In the next step, the beads were added to 400 µl of an elution buffer (25 mM acetate buffer at pH 3.0) and shaken for one hour at room temperature. The supernatant was then removed from the beads and the beads were cleaned by washing. Both the bound and eluted IgG was an analysed by an ELISA assay. The results of these investigations are shown in the following Table 1.

**Table 1**

| Peptide | IgG bound [µg] | IgG Elution [µg] | Recovery [IgG] |
|---|---|---|---|
| Peptide 1 | 45 | 41 | 91% |
| Peptide 2 | 55 | 52 | 95% |
| Peptide 3 | 60 | 55 | 92% |

This Example shows, that IgG can selectively be bound to an oligopeptide immobilized on a latex carrier material and eluted from this carrier material to provide a pure product.

### Example 2: Indirect detection of peptide binding to IgG with albumin control

In a first step, IgG and albumin, respectively, were attached to an ELISA plate. The peptide 3 of Example 1 was then added to the coated ELISA plate. The peptide was dissolved in three buffers comprising PBS, PBS and 0,1 % Tween and PBS, 0,1 % Tween and 3% Gelafusal. The peptide was added in a dilution series and was diluted 1:4 each. For detecting the binding of the biotinylated peptide, streptavidine-HRP was added. The substrate reaction was then started with TMB Seramun until a coloration became visible. The substrate reaction was stopped by the addition of sulphuric acid and the ELISA plate was measured at 492 and 629 nm, respectively, to obtain the results.

The log c (peptide) to the measured absorption shows an increase of the binding of a peptide 3 to IgG on increase of the peptide 3 concentration in tween containing buffers. Albumin in contrast does not show an effect as all concentration as well as the control exhibited about the same level of absorption. This is indicative unspecific binding of the conjugate streptavidine-HRP to the albumin.

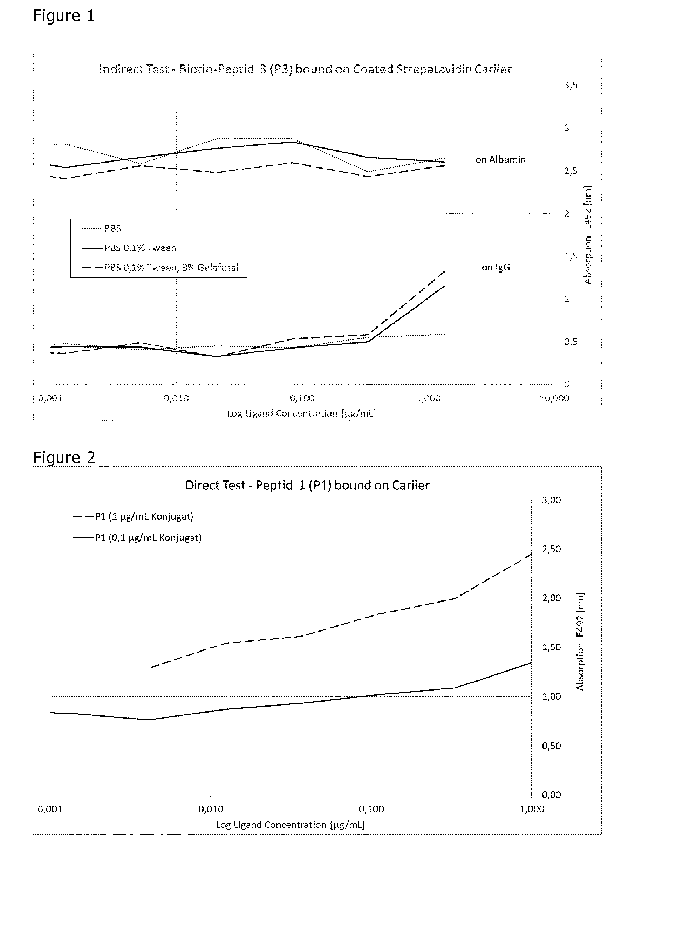

The results of these measurements are illustrated in Figure 1.

### Example 3: Direct detection of the binding of a peptide to IgG:

In a first step, an ELISA plate was coated with a ligand of any one of peptides 1 to 3. The peptide was applied as a dilution series and was diluted 1:3 each. A blank not containing the peptide was used as a control. In a second step, IgG was applied in a constant amount to the plates followed by anti-human IgG-HRP. A substrate reaction was started with TMB Seramun until a coloration became visible. The reaction was stopped by addition of sulphuric acid and subsequently, the ELISA plate was measured at 492 and 629 nm to obtain the results.

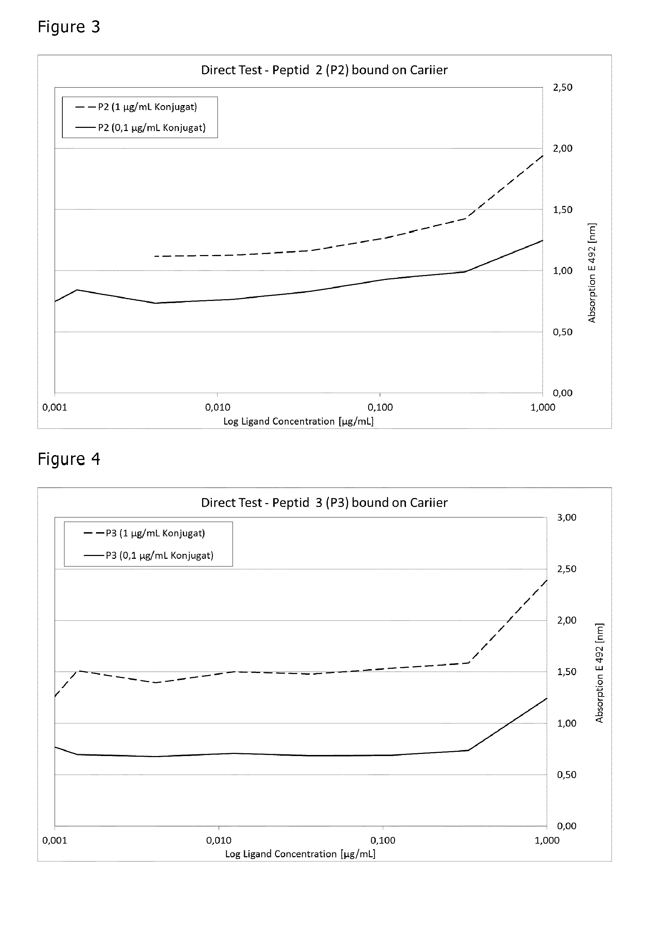

The Log c (peptide) to the measured absorption shows an increase of the binding of IgG to the peptide 1 to peptide 3 at an increasing ligand concentration. The results of the investigations are provided in Figures 2 through 4.

## Claims

1. Sorbent for binding proteins from blood plasma, comprising a solid carrier material and an oligopeptide immobilized thereon, which selectively binds a protein from the blood plasma.

2. Sorbent according to claim 1, wherein the oligopeptide has 8 to 50, preferably 10 to 30 and in particular 12 to 20 amino acids.

3. Sorbent according to claim 1 or 2, wherein the oligopeptide selectively binds to IgG, factor V, factor VIII or albumin.

4. Sorbent according to claim 2, wherein the oligopeptide comprises a sequence selected from
i) KEDGNKPGKED
ii) KEDGNKP
iii) DTVNDIAKANGTTAD
iv) LETEKQISEASRKSL
v) DLTAAAVADTVA
vi) DAPTEPEKPE
vii) KQISD/EASR
viii) LEKLELDYLKKL
ix) SQTPDTKPGNK
x) LSRDLEASRAA
or a sequence having at least 80 % sequence identity with the above indicated i) to x) and having a binding affinity toward IgG, wherein D/E indicates that the amino acid is either D or E.

5. Sorbent according to claim 2, wherein the oligopeptide comprises a sequence selected from
i) LEGYDLRRWEKWE
ii) HNYGVYTKVSRYL
iii) GDSGGPMVASFHG
iv) FVHPNYSKSTTDNDIALLH
v) TGWGYHSSREKEA
or a sequence having at least 80 % sequence identity with the above indicated i) to v) and having a binding affinity towards factor V or factor VIII.

6. Sorbent according to any one of claims 1 to 5, wherein the solid carrier material is a latex, preferably a magnetic latex.

7. Container having an inlet and an outlet for fluids which comprises a filling of a sorbent according to any one of claims 1 to 6.

8. Container according to claim 7, wherein the outlet is positioned on the side opposite to the inlet on the container.

9. Oligopeptide having 8 to 50, preferably 10 to 30 and in particular 12 to 20 amino acids which comprises a sequence selected from
i) KEDGNKPGKED
ii) KEDGNKP
iii) DTVNDIAKANGTTAD
iv) LETEKQISEASRKSL
v) DLTAAAVADTVA
vi) DAPTEPEKPE
vii) KQISD/EASR
viii) LEKLELDYLKKL
ix) SQTPDTKPGNK
x) LSRDLEASRAA
xi) LEGYDLRRWEKWE
xii) HNYGVYTKVSRYL
xiii) GDSGGPMVASFHG
xiv) FVHPNYSKSTTDNDIALLH
xv) TGWGYHSSREKEA
or a sequence having at least 80 % sequence identity with the above indicated i) to xv) and having a binding affinity toward IgG, factor V or factor VIII, wherein D/E indicates that the amino acid is either D or E.

10. Use of the oligopeptide of claim 9 for the detection of the presence or absence of IgG, in particular specific IgG, factor V or factor VIII in an analyte.

11. Method for separating a blood plasma protein from blood plasma, supernatants of cell cultures or protein preparations comprising the steps of
i) bringing the blood plasma, supernatants of cell cultures or protein preparations in contact with a sorbent according to any one of claims 1 to 6 under conditions which allow for binding of at least one protein from the blood plasma to the sorbent,
ii) separating the sorbent from the blood plasma, supernatants of cell cultures or protein preparations and
iii) subsequently separating the bound protein from the sorbent.

12. Method according to claim 11, wherein the sorbent is subjected to washing after step ii) and before step iii) under conditions where the protein remains bound to the sorbent.

13. Method for separating an autologous protein from blood plasma comprising the steps of
i) conveying blood from a donor to a first separation device, which separates the blood plasma from the blood,
ii) conveying the blood plasma to a second separation device, at which it is brought in contact with a sorbent according to any one of claims 1 to 6 under conditions which allow for binding of at least one protein from the blood plasma to the sorbent, and
iii) subsequently collecting the blood plasma in a container.

14. Method for separating an autologous protein from blood plasma comprising the steps of
i) conveying blood from a donor to a first separation device, which separates the blood plasma from the blood,
ii) conveying the blood plasma to a second separation device, at which it is brought in contact with a sorbent according to any one of claims 1 to 6 under conditions which allow for binding of at least one protein from the blood plasma to the sorbent, and
iii) subsequently returning at least a portion of the blood plasma to the donor.

15. Method for depleting an autologous protein in blood plasma comprising the steps of
i) conveying blood from a patient to a first separation device, which separates the blood plasma from the blood,
ii) conveying the blood plasma to a second separation device, at which it is brought in contact with a sorbent according to any one of claims 1 to 6 under conditions which allow for binding of at least one protein from the blood plasma to the sorbent, and
iii) subsequently returning at least a portion of the blood plasma to the donor,
wherein the patient suffers from an overproduction of the protein to be removed in step ii).

16. The method according to any one of claims 13 to 15, wherein the sorbent is subjected to conditions to allow for release of the bound protein in an online manner.

17. The method according to any one of claims 13 to 15, wherein the blood plasma is brought in contact with different sorbents according to claims 1 to 7, which are arranged in a consecutive fashion.
